# EUROPEAN PATENT SPECIFICATION

(11) **EP 4 539 793 B1**
(45) Date of publication and mention of the grant of the patent: **18.11.2026**
(21) Application number: 23731772.2
(22) Date of filing: 14.06.2023
(51) Int. Cl.: A61F 9/00

(54) **INJECTOR DEVICE**
INJEKTORVORRICHTUNG
DISPOSITIF INJECTEUR

(30) Priority: 20.06.2022 NL 2032219
(43) Date of publication of application: 23.04.2025
(73) Proprietor: Crea IP B.V., 3237 MG Vierpolders (NL)
(72) Inventor: ANGELINI, Giov Battista, 00015 Monterotondo (IT); HIEMSTRA, Joep, 3237 MG Vierpolders (NL); CLEIJ, Mervin Gerard, 3052 PV Rotterdam (NL); BOUKHNY, Mikhail, Irvine, California 92677 (US); PERNICE, Giovanni, 23807 Merate (IT)
(74) Representative: Nederlandsch Octrooibureau
(86) International application number: PCT/NL2023/050332
(87) International publication number: WO 2023/249485

(56) References cited:
- WO-A1-2017/218610
- US-A- 4 722 366

## Description

### Field of the invention

The present invention relates to an injector for ophthalmic delivery. The present invention further relates to a system comprising such an injector and a control unit.

### Background art

Patent publication WO2017/218610A1 describes an injector for subretinal delivery of therapeutic agent, having two fluid supply ducts for the delivery of bleb fluid and therapeutic agent. The fluid ducts are connected to a valve assembly that includes actuation arms that can be operated to switch the valve.

The injector of the prior art therefore requires an active user intervention for closure of the valve assembly in order to block the flow of the bleb fluid during injection of the therapeutic agent to a patient's eye, and vice versa. The transparent fluids also provide no indication for the user of when to actuate the valve. Often, delays in the actuation of the valve will affect the composition of the delivery.

The fluid that has not been delivered to the eye remains in the injector downstream of the valve, and will be mixed with the agent when the agent is being delivered, and vice-versa.

Consequently control on the content of and the amount of the delivery with the injector of the prior art is relatively low.

It is an object of the invention to provide an injector for gas and fluid for ophthalmic applications that provides for a quick and safe liquid and gas irrigation of the eye during surgery. It is a further object of the invention to provide a system comprising the injector and a control unit that provides a highly controlled delivery of gas and fluid to the eye during surgery.

### Summary of the invention

According to a first aspect, the invention provides an injector for use in ophthalmic applications, having a gas supply tube and a liquid supply tube, both supply tubes being interconnected at a junction member that is in fluid communication with an outflow cannula, characterised in that a selective flow member is placed in the gas supply tube, near the junction member, allowing gases to pass through and blocking the passage of liquid. The injector of the invention allows a simplified and controlled delivery of liquid and gas to the sensitive human eye, during surgery. Gas, in particular, may flow in two directions through the gas supply tube because the selective flow member only blocks liquid. This bidirectional gas flow provides for the possibility of both gas delivery to the eye and aspiration of an excess of gas in the tube in the opposite direction. Liquid is blocked by the selective flow member, forming a liquid buffer of a small volume in the gas supply tube. This buffer is formed in the injector to prevent the delivery of any gas into the eye when only liquid should be delivered. This buffer can be created in a passive manner in the injector of the invention, it does not require user intervention. Furthermore the volume of the buffer is controlled by the position of the selective member along the gas supply tube since liquid cannot pass through the selective member. As a result, the volume of the buffer will remain sufficiently small to be removed when switching back to a gas delivery. The switching between liquid and gas delivery is therefore accelerated as the volume of liquid between the selective flow member and the junction member which is required as a buffer but needs to be removed prior to gas delivery, can be minimized. Therefore the switch between liquid and gas irrigation of the eye is quickened. Excess gas can also be directly reinjected/aspirated back into a or in direction of a gas supply as gas may flow through the selective flow member, which results in an improved intraocular pressure, during liquid delivery.

In a further embodiment the selective flow member consists of a membrane comprising polytetrafluoroethylene, PTFE, the membrane having a maximum pore size between 0.2 and 1.0 µm. The selective flow member made of PTFE forms a highly hydrophobic membrane that is sufficiently porous in order to provide for a passage of gas, while blocking a flow of liquid through the use of relatively small pores. The membrane further provides for bacterial retention, by blocking bacteria's with a size above the maximum pore size. As a result only a clean air flow is irrigated into the eye.

In a further embodiment, the PFTE membrane has a thickness between 0.01 and 1 mm. Using an hydrophobic material that is relatively thin allows most of the gas to pass through it. The membrane therefore does not affect a flow rate of gas during gas delivery. Time of gas delivery is unaffected.

In a further embodiment a selective flow member being placed in the gas supply tube near the junction member so that at most 1.5 mL is fillable with liquid, preferably at most 0.5 mL, more preferably at most 0.1 mL. A buffer volume controlled to be less than 1.5 mL quickens a removal of the liquid buffer when gas is to be delivered into the eye. In that way switching from a liquid delivery mode to a gas delivery mode is faster, while ensuring enough gas is present in the tube ready for delivery without need for immediate further supply from an external unit.

In a further embodiment the selective flow member is placed in the gas supply tube in a chamber, the chamber having a first segment and a second segment, the second segment nearest to the junction member, each of the first segment and the second segment having an opening in its surface, the selective flow member separating the chamber in a first compartment near the first segment and a second compartment near the second segment, each of the first compartment and second compartment in fluid connection with the gas supply tube via the opening, liquid in the second compartment blocked from entering the first compartment. The selective flow member maintained in the chamber may be in contact with a volume of both liquid and gas. The chamber provides for a compact design of the injector, allowing a buffer of liquid to form in the chamber for preventing gas delivery during liquid injection.

In a further embodiment each of the first segment and second segment having on its surface a connector extending radially to the opening to receive the gas supply tube, the gas supply tube in fluid connection with the first compartment and the second compartment of the chamber via the opening. The gas supply tube may be connected to the chamber via the connectors while possible bends and twists are reduced at the tube, in a radial direction to the surface of the chamber.

In a further embodiment, the first segment and the second segment are integral elements of the chamber. By providing a single element chamber a maximum sealing of the content of the chamber is provided.

In a further embodiment the first segment and the second segment being releasably connected. The releasable connection of the chamber parts makes it possible to open the chamber for cleaning and/or changing the membrane of the selective flow member.

In a further embodiment the opening in the surface of the first segment and the opening in the surface of the second segment being substantially aligned along a main axis of the gas supply tube. A more compact design of the injector is provided by the alignment of the openings with a main direction of the gas supply tube.

In a further embodiment the chamber has a volume of 1 mL, preferably of 0.3 mL, more preferably of 0.1 mL. The chamber has small dimensions which offers easy handling to a user, eg surgeon, while still offering sufficient volume of liquid to prevent gas delivery during liquid injection.

In a further embodiment the chamber has a round shape, such as a disc-shape or a spherical shape. The chamber with a round shape provides for a user friendly smooth entity which may be hold in a hand.

In a further embodiment the fluid connection between the gas supply tube and the opening comprising one or more Luer connectors. Fluid-tight connections between the tube and the interior of the chamber are provided by the use of such additional connector elements.

In a second aspect the invention provides a system comprising a control unit, a gas supply, and a liquid supply, each of the gas supply and the liquid supply connected to a gas supply tube and a liquid supply tube, respectively, of an injector according to any of the preceding claims, the control unit being adapted to: activate the gas supply to push up gas into the gas supply tube, past the selective flow member at a gas pressure X and supply gas to the outflow cannula, and switch to a supply of liquid through the outflow cannula by flowing liquid up to the selective flow member at a liquid pressure higher than the gas pressure X, and supplying liquid to the outflow cannula. The system of the invention provides for a controlled outflow, with an improved intraocular pressure due to excess gas that may be aspired back in the direction of the gas supply at liquid delivery. The system allows for a quick switching from liquid to gas flow, and vice versa, thanks to sufficient gas pressure control during injection and aspiration.

In a further embodiment the control unit being further adapted to, at the step of activating the gas supply, activate the liquid supply and push up liquid past the liquid supply tube so as to form a liquid to gas junction forms in the liquid supply tube. Further control at gas delivery may be provided by the system, where a gas buffer is created in the liquid supply tube by accurate control of the control unit of the pressure of both gas and liquid.

In a further embodiment the control unit adapted to control the liquid pressure and the gas pressure in each supply tube in a range of 0 to 200 mmHg. Via accurate control of liquid and gas pressure over a broad pressure range, any of the liquid or the gas can be either injected into the outflow cannula or aspired in direction of an ophthalmic unit, by applying varying pressures.

### Brief description of the drawings

The present invention will be discussed in more detail below, with reference to the attached drawings, in which,
Figure 1A shows a schematic view of an injector according to the invention.
Figure 1B shows a schematic view of an injector according to the invention, upon liquid delivery to an eye.
Figure 1C shows a schematic view of an injector according to the invention, during gas injection.
Figure 2 shows a perspective view of an injector according to an embodiment of the invention.
Figure 3 shows an exploded view of the disc-shaped chamber of the injector according to the embodiment of Figure 2.
Figure 4 shows a perspective view of an injector according to another embodiment of the invention.
Figure 5 shows an exploded view of the disc-shaped chamber of the injector according to the embodiment of Figure 4.
Figure 6 shows a flowchart of a system according to the invention.

### Detailed description

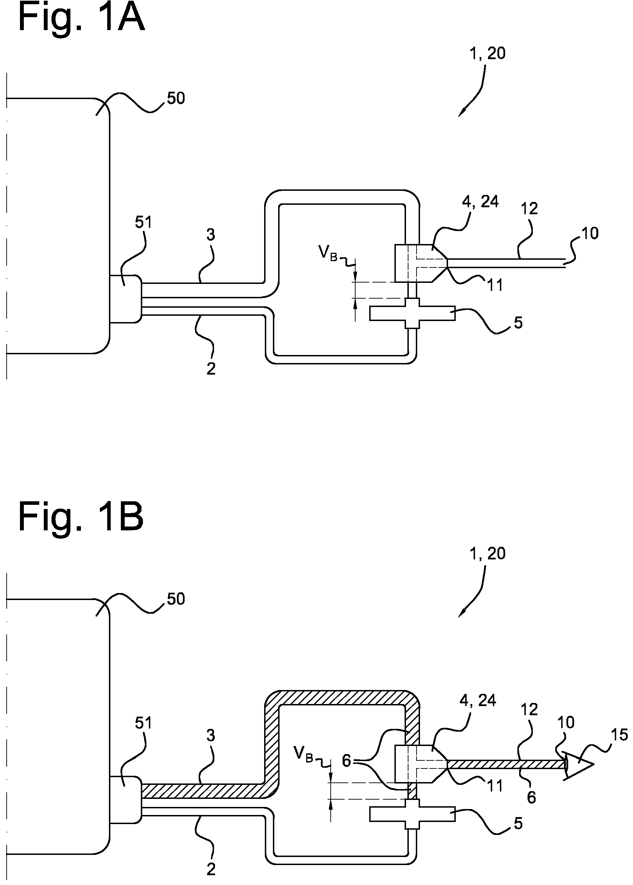

Fig. 1A shows a schematic view of an injector 1, 20 for ophthalmic delivery according to the invention. The injector 1, 20 comprises a gas supply tube 2, a liquid supply tube 3, a junction member 4, 24, a selective flow member 5 and an outflow cannula 12. The gas supply tube 2 and the liquid supply tube 3 both serve as inlet tube of the device, for gas such as air and liquid such as for example water or a cleansing solution, BSS, respectively, to be delivered into an eye. The injected gas and liquid help sustain sufficient irrigation of the eye, for instance at the subretinal level, during surgery, and/or ensure a post-surgery protection and healing. As shown, the gas and liquid are input to each of the supply tubes 2, 3 of the injector from an ophthalmic unit 50 to which the tubes 2, 3 may be connected via a connector 51. The connector 51 as shown connects both gas and liquid supply tubes 2, 3 to the ophthalmic unit 50. In other embodiments falling within the scope of the invention (not shown) there may be a first connector connecting the gas supply tube 2 to the ophthalmic unit 50, and a second connector, different from the first connector, connecting the liquid supply tube 3 to the ophthalmic unit 50. The ophthalmic unit 50 corresponds to a working station and may further include any of electronics, working arrangements and supplies. It is noted that the connector 51 may be any connector known to the person skilled in the art that will provide a fluid-tight connection between the injector 1, 40 and the ophthalmic unit 50. The same applies to each of the separate first and second connectors. The junction member 4, 24 is a connecting element of the injector 1, 20 connecting the gas supply tube 2, the liquid supply tube 3 and the outflow cannula 12. The junction member in the view shown is T-shaped and connects both inlet supply tubes 2, 3 to the outflow cannula 12. It will be understood that other embodiments comprising a differently shaped junction, such as a Y-shaped junction also falls within the scope of the invention. The junction member 4, 24 is open to the passage of liquid from the liquid supply tube 3 or gas from the gas supply tube 2. The outflow cannula 12 extends from the proximal end 11 of the junction member 4, 24 and allows an outflow of either gas or liquid from the supply tubes at its distal end 10. The outflow cannula 12 has, at its distal end 10, an outlet that can be placed at or in the vicinity of an eye for delivery of gas or liquid to the eye. The selective flow member 5 is connected to the gas supply tube 2 at a distance from the junction member 4, 24. This distance corresponds to a volume V_{B} of the gas supply tube 2. The selective flow member 5 may be both an integral part of the gas supply tube 2 or may be attached to a gas supply tube 2 by a user. The selective flow member 5 serves to block liquid flow into the gas supply tube 2 in direction of the ophthalmic unit 50. However the selective flow member 5 allows a bidirectional flow of gas through it. The selective flow member 5 can be formed by a hydrophobic filter. The liquid can occupy the volume V_{B} of the gas supply tube 2 that is delimited by the location of the selective flow member 5. This volume of buffer liquid in the gas supply tube 2 ensures that no gas enters the eye during liquid delivery. The selective flow member 5 will be described in more detail in Fig. 1B-1C during liquid delivery and gas delivery, respectively.

Figure 1B shows a schematic view of the injector 1, 20 according to the invention upon delivery of a liquid 6. The outlet of the outflow cannula 12 at the distal end 10 has been positioned into an eye 15. The liquid 6, such as a BSS solution, is first discharged from the ophthalmic unit 50 into the liquid supply tube 3. During liquid delivery, pressure in the gas supply tube 2 is lower than the pressure in the liquid supply tube 3. The discharge and reinjection/aspiration of liquid and gas from/to the ophthalmic unit 50 may be at least partially controlled by an electronic control unit (not shown) within the ophthalmic unit 50. Upon discharge of liquid 6, the liquid 6 fills the liquid supply tube 3 through the junction member 4, 24 and enters partly into the gas supply tube 2 over the distance between the junction member 4, 24 and the selective flow member 5. In this way, the liquid buffer in the gas supply tube 2 of volume V_{B} is formed. As described earlier, the selective flow member 5 blocks the liquid 6 from filling the gas supply tube 2 beyond the selective flow member 5 in the direction of the ophthalmic unit 50. In that way the buffer of volume V_{B} is passively formed.

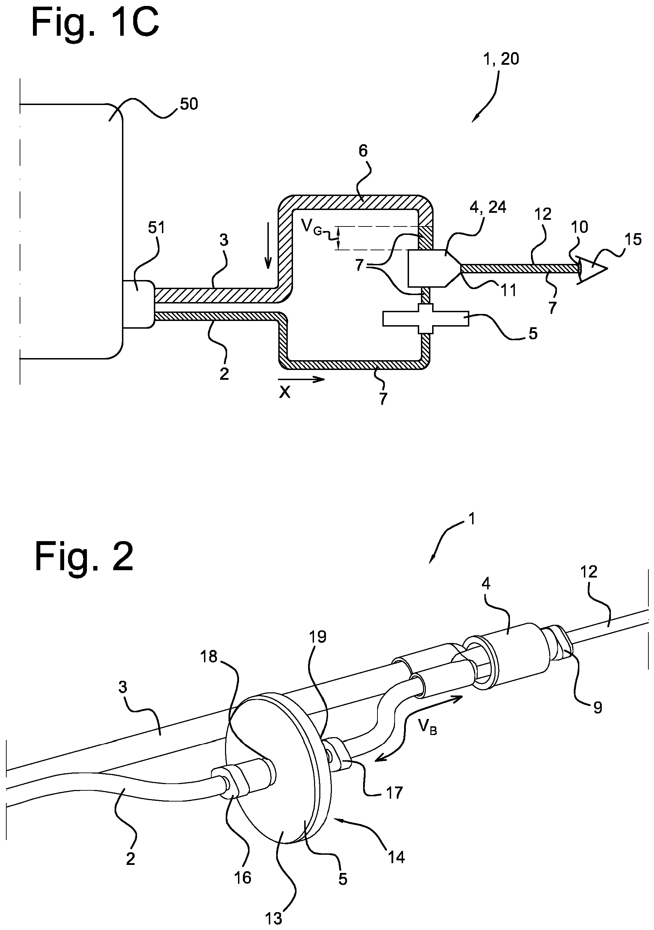

Figure 1C shows a schematic view of the injector 1, 20 according to the invention during delivery of a gas 7. The outlet of the outflow cannula 12 at the distal end 10 has been positioned into the eye 15. In this mode, gas 7 is discharged from the ophthalmic unit 50 at a pressure X into the gas supply tube 2.The gas 7 can flow through the selective flow member 5. The gas may also be in a preferred embodiment optionally discharged in a portion of the gas supply tube 2, filling a volume V_{G} in the liquid supply tube 3, that is free of liquid. A buffer of gas may be therefore optionally formed in the liquid supply tube 3, avoiding any liquid 6 from being delivered in the eye 15. Furthermore, via the outflow cannula 12, gas can be delivered into the eye 15. When switching back to supplying liquid in Fig. 1B the gas 7 may be first aspired into the ophthalmic unit 50 as it flows through selective flow member 5, thus freeing up the buffer volume V_{B}, which can be filled by the liquid 6 again.

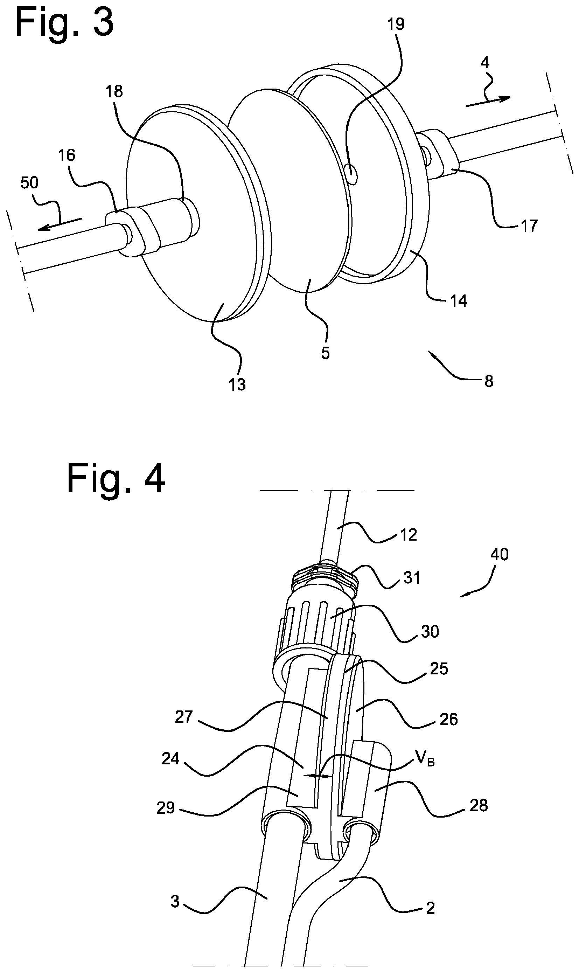

Figure 2 shows a perspective view of an injector 1 according to an embodiment of the invention. The injector 1 comprises, as shown in Fig. 1A-1C a gas supply tube 2, a liquid supply tube 3, a junction member 4, an outflow cannula 12 and a selective flow member (not shown). The selective flow member 5 is shown in the exploded view of Fig. 3. The selective flow member 5 is located within a disc-shaped chamber 8 and is placed at a distance from the junction member 4 in the liquid supply tube 2 allowing for a buffer volume V_{B}. The shown disc-shaped chamber 8 comprises two circular portions or segments 13, 14 that are interconnected. In alternative embodiment the chamber 8 may as well have a different shape, such as a rectangular or squared shape. Each segment 13, 14 is on its outer surface connected to the gas supply tube 2 via an opening 18, 19 on each side of the chamber 8 in the center of the disc. The openings 18, 19 may be circular. It is noted that other embodiments with an opening of any shape, and not centered also fall within the scope of the invention. The liquid tight connection between the gas supply tube 2 and the selective flow member 5 at each opening 18, 19 may be facilitated by means of one or more Luer connectors 16, 17. The same applies to the tight junction between the junction member 4 and the outflow cannula 12, which may comprise a Luer connector 9. It is noted that alternative embodiments falling within the scope of the invention may comprise other connectors and/or have been manufactured in ways providing a fluid tight connection between the two elements.

Figure 3 shows an exploded view of the disc-shaped chamber 8 of the injector 1 according to the embodiment of Figure 2. The segments 13, 14 enclose the selective flow member 5. The selective flow member 5 comprises a membrane of selective flow material. The membrane of selective flow material separates the volume of the disc-shaped chamber 8 into a first compartment and a second compartment: a gas compartment in direction of the ophthalmic unit 50 and a liquid buffer compartment on the side of the junction member 4. The membrane of selective flow material comprises polytetrafluoroethylene PTFE which is a highly hydrophobic or water repellent material. The selective flow membrane comprising PTFE further incorporates micropores having a maximum pore size in the range in the range of 0.2 to 1.0 µm. A thickness of the membrane is preferably in the range of 0.01 to 1 mm. This selective flow sheet of PTFE allows an high gas flow through the sheet from the gas chamber to the liquid chamber. However the same sheet blocks the flow of water and water-based solutions such as BSS through the sheet from the liquid chamber to the gas chamber, according to the description in Fig. 1A-1C.

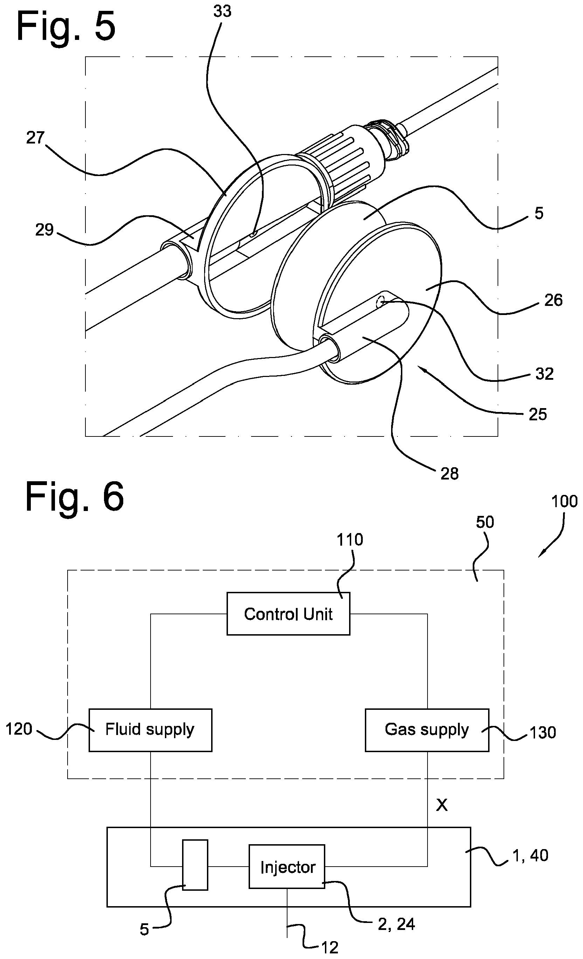

Figure 4 shows a perspective view of an injector 40 according to another embodiment of the invention. The injector 40 comprises, as shown in Fig. 1A-1C a gas supply tube 2, a liquid supply tube 3, a junction member 24, an outflow cannula 12 and a selective flow member (not shown). The selective flow member 5 is shown in the exploded view of Fig. 5. The selective flow member 5 is located within a disc-shaped chamber 25 and near the junction member 24, in the liquid supply tube 2 allowing for a buffer volume V_{B}. The shown disc-shaped chamber 25 comprises two circular portions or segments 26, 27 that are interconnected. In other embodiments the chamber may as well have a different shape, such as a rectangular or squared shape with corresponding segments. Each segment 26, 27 comprises a connector 28, 29 extending radially on its surface to receive the gas and liquid supply tubes 2, 3, respectively. The connector 29 also contains a portion of the gas tube 2 connecting to the liquid supply tube 3 at a junction member 24 (not shown) inside the connector 29. In the connector 28, 29 holding it, the gas supply tube 2 connects to a central opening 32, 33 on each segment of the chamber, respectively. The selective flow member 5 may further fluidly connected to the outflow cannula 12 via an additional joint member 30. The junction between the junction member 24 and the outflow cannula 12, may also comprise a Luer connector 31. It is noted that alternative embodiments falling within the scope of the invention may comprise other connectors and/or have been manufactured in ways providing a fluid tight connection between two elements.

Figure 5 shows an exploded view of the disc-shaped chamber 25 of the injector 40 according to the embodiment of Figure 4. The device is shown from a new angle that allows a better view of the selective flow member 5. The segments 26, 27 enclose the selective flow member 5. The selective flow member 5 is formed by a membrane of selective flow material. The membrane of selective flow material separates the volume of the disc-shaped chamber 25 into a gas compartment near segment 26, and a liquid buffer compartment in direction of the outflow cannula 12 near segment 27. The opening 32, 33 in the chamber segments is circular as illustrated in Fig. 5 but may as well have a different shape. The opening may as well at another location on each portion of the chamber, shifted from a center of the chamber 25. The fluid tight connection between the gas supply tube 2 and the disc-shaped chamber 25 at each opening 32, 33 may be facilitated by means of one or more Luer connectors within the connectors (not shown). It is noted that alternative embodiments falling within the scope of the invention may comprise other connectors and/or have been manufactured in ways providing a fluid tight connection between the two elements. The sheet of selective flow material comprises PTFE which is a highly hydrophobic or a water repellent material. The selective flow sheet comprising PFTE having a maximum pore size in the range in the range of 0.2 to 1.0 µm. A thickness of the membrane is preferably in the range of 0.01 to 1 mm. This selective flow sheet of PTFE allows an high gas flow through the sheet from the gas chamber to the liquid chamber. However the same sheet blocks the flow of water and water-based solutions such as BSS through the sheet from the liquid chamber to the gas chamber, according to the description in Fig. 1A-1C.

Figure 6 shows a schematic representation of a system 100 according to the invention. The system comprises a control unit 110, a liquid supply 120 and a gas supply 130. The control unit 110 may assist and/or fully control in operation the discharge and refill of each of the liquid supply 120 and the gas supply 130 to and from liquid and gas supply tubes of an injector device 1, 40 respectively. At least one of the control unit 110 and the liquid and gas supplies 120, 130 may be part of an ophthalmic unit 50, preferably all are part of the ophthalmic unit 50. In other embodiments the ophthalmic unit comprises at least one of a liquid supply, gas supply or control unit, while the other elements are separate entities. The control unit 110 controls the liquid supply and the gas supply in fluid connection with an injector device 1, 40 via valves (not shown) near the liquid and gas supplies 120, 130, the injector device being one of Fig. 1, 2 or 4. The valves in combination with provided pressures by the supplies provide the control of the system. The control unit may control the gas to be delivered to the injector device at a pressure X. The control unit may further be used for aspiration of the liquid and/or gas in the respective supply tubes of the injector back into their respective supplies at switching from one mode to the other mode (liquid-gas and vice versa), by applying a liquid pressure. Pressures in the supply tubes generally do not exceed 200 mmHg, preferably do not exceed 160 mmHg. The selective flow member 5 of the injector 1, 40 ensures that no liquid may be aspired up into the gas tube and gas supply of the ophthalmic unit.

## Claims

1. Injector (1, 40) for use in ophthalmic applications, having a gas supply tube (2) and a liquid supply tube (3), both supply tubes being interconnected at a junction member (4, 24) that is in fluid communication with an outflow cannula (12),
**characterised in that** a selective flow member (5) formed by a hydrophobic filter is placed in the gas supply tube (2) at a distance from the junction member (4, 24) corresponding to a volume V_{B} of the gas supply tube fillable with liquid, allowing gases to pass through and blocking the passage of liquid.

2. Injector (1, 40) according to claim 1, the selective flow member (5) consisting of a membrane comprising polytetrafluoroethylene, PTFE, the membrane having a maximum pore size between 0.2 and 1.0 µm.

3. Injector (1,40) according to claim 2, the membrane having a thickness between 0.01 and 1 mm.

4. Injector (1, 40) according to any of the preceding claims, wherein the volume V_{B} is at most 1.5 mL, preferably at most 0.5 mL, and more preferably at most 0.1 mL.

5. Injector (1, 40) according to any of the preceding claims, the selective flow member (5) being placed in the gas supply tube (2) in a chamber (8, 25), the chamber (8, 25) having a first segment (13, 26) and a second segment (14, 27), the second segment (14, 27) nearest to the junction member (4, 27), each of the first segment (13, 26) and the second segment (14, 27) having an opening (18, 19, 32, 33) in its surface, the selective flow member (5) separating the chamber (8, 25) in a first compartment near the first segment (13, 26) and a second compartment near the second segment (14, 27), each of the first compartment and second compartment in fluid connection with the gas supply tube (2) via the opening (18, 19, 32, 33), liquid in the second compartment blocked from entering the first compartment.

6. Injector (1, 40) according to claim 5, each of the first segment and second segment (26, 27) having on its surface a connector (28, 29) extending radially to the opening (32, 33) to receive the gas supply tube (2), the gas supply tube (2) in fluid connection with the first compartment and the second compartment of the chamber (25) via the opening (32, 33).

7. Injector (1, 40) according to any of claims 5 or 6, the first segment (13, 26) and the second segment (14, 27) being integral elements of the chamber (8, 25).

8. Injector (1, 40) according to any of claims 5 or 6, the first segment (13, 26) and the second segment (14, 27) being releasably connected.

9. Injector (1, 40) according to any of claims 5 to 7, the opening (18, 32) in the surface of the first segment (13, 26) and the opening (19, 33) in the surface of the second segment (14, 27) being substantially aligned along a main axis of the gas supply tube (2).

10. Injector (1, 40) according to any of claims 5 to 9, the chamber (8, 25) having a volume of 1 mL, preferably 0.3 mL, more preferably 0.1 mL.

11. Injector (1, 40) according to any of claims 5 to 10, the chamber (8, 25) having a round shape, such as a disc-shape or a spherical shape.

12. Injector (1, 40) according to any of claims 5 to 11, the fluid connection between the gas supply tube (2) and the opening (18, 19, 32, 33) comprising one or more Luer connectors (16, 17).

13. System (100) comprising a control unit (110), a gas supply (120), and a liquid supply (130), each of the gas supply (120) and the liquid supply (130) connected to a gas supply tube (2) and a liquid supply tube (3), respectively, of an injector (1, 40) according to any of the preceding claims, the control unit (110) being adapted to:
- activate the gas supply (120) to push up gas into the gas supply tube (2), past the selective flow member (5) at a gas pressure X and supply gas to the outflow cannula (12), and
- switch to a supply of liquid (130) through the outflow cannula (12) by flowing liquid up to the selective flow member (5) at a liquid pressure higher than the gas pressure X, and supplying liquid to the outflow cannula (12).

14. System (100) according to claim 13, the control unit being further adapted to, at the step of activating the gas supply (120), activate the liquid supply (130) and push up liquid past the liquid supply tube (3) so as to form a liquid to gas junction in the liquid supply tube (3).

15. System (100) according to claim 13 or claim 14, the control unit adapted to control the liquid pressure and the gas pressure in each supply tube (2,3) in a range of 0 to 200 mmHg.

## Patentansprüche

1. Injektor (1, 40) zur Verwendung in augenheilkundlichen Anwendungen, wobei dieser ein Gaszuführrohr (2) und ein Flüssigkeitszuführrohr (3) hat, wobei beide Zuführungsrohre an einem Verbindungselement (4, 24) miteinander verbunden sind, das in fluidischer Verbindung mit einer Abflusskanüle (12) ist, **dadurch gekennzeichnet, dass**
ein Auswahldurchflusselement (5), das durch einen hydrophoben Filter gebildet ist, in dem Gaszuführrohr (2) in einem Abstand von dem Verbindungselement (4, 24) platziert ist, der einem Volumen V_{B} des Gaszuführrohrs entspricht, das mit Flüssigkeit befüllbar ist, wobei dieses Gasen ermöglicht hindurch zu treten und den Durchgang von Flüssigkeit blockiert.

2. Injektor (1, 40) nach Anspruch 1, wobei das Auswahldurchflusselement (5) aus einer Membran besteht, die Polytetrafluorethylen, PTFE, aufweist, wobei die Membran eine maximale Porengröße zwischen 0,2 und 1,0 µm hat.

3. Injektor (1, 40) nach Anspruch 2, wobei die Membran eine Dicke zwischen 0,01 und 1 mm hat.

4. Injektor (1, 40) nach einem der vorhergehenden Ansprüche, wobei das Volumen V_{B} höchstens 1,5 ml, bevorzugt höchstens 0,5 ml, und besonders bevorzugt höchstens 0,1 ml ist.

5. Injektor (1, 40) nach einem der vorhergehenden Ansprüche, wobei das Auswahldurchflusselement (5) in dem Gaszuführrohr (2) in einer Kammer (8, 25) platziert ist, wobei die Kammer (8, 25) ein erstes Segment (13, 26) und ein zweites Segment (14, 27) hat, wobei das zweite Segment (14, 27) am nächsten zu dem Verbindungselement (4, 27) ist, wobei jedes des ersten Segments (13, 26) und des zweiten Segments (14, 27) eine Öffnung (18, 19, 32, 33) in seiner Oberfläche hat, wobei das Auswahldurchflusselement (5) die Kammer (8, 25) in ein erstes Abteil nahe dem ersten Segment (13,26) und einem zweiten Abteil nahe dem zweiten Segment (14, 27) trennt, wobei jedes des ersten Abteils und zweiten Abteils mit dem Gaszuführrohr (2) durch die Öffnung (18, 19, 32, 33) in fluidischer Verbindung ist, wobei Flüssigkeit in dem zweiten Abteil am Eintreten in das erste Abteil gehindert ist.

6. Injektor (1, 40) nach Anspruch 5, wobei jedes des ersten Segments und zweiten Segments (26, 27) auf seiner Oberfläche einen Verbinder (28, 29) hat, der sich radial zu der Öffnung (32, 33) erstreckt, um das Gaszuführrohr (2) aufzunehmen, wobei das Gaszuführrohr (2) mit dem ersten Abteil und dem zweiten Abteil der Kammer (25) durch die Öffnung (32, 33) in fluidischer Verbindung ist.

7. Injektor (1, 40) nach einem der Ansprüche 5 oder 6, wobei das erste Segment (13, 26) und das zweite Segment (14, 27) integrale Bauteile der Kammer (8, 25) sind.

8. Injektor (1, 40) nach einem der Ansprüche 5 oder 6, wobei das erste Segment (13, 26) und das zweite Segment (14, 27) lösbar verbunden sind.

9. Injektor (1, 40) nach einem der Ansprüche 5 bis 7, wobei die Öffnung (18, 32) in der Oberfläche des ersten Segments (13, 26) und die Öffnung (19, 33) in der Oberfläche des zweiten Segments (14, 27) im Wesentlichen entlang einer Hauptachse des Gaszuführrohrs (2) ausgerichtet sind.

10. Injektor (1, 40) nach einem der Ansprüche 5 bis 9, wobei die Kammer (8, 25) ein Volumen von 1 ml, bevorzugt 0,3 ml, besonders bevorzugt 0,1 ml hat.

11. Injektor (1, 40) nach einem der Ansprüche 5 bis 10, wobei die Kammer (8, 25) eine runde Form hat, wie beispielsweise eine Scheibenform oder eine Kugelform.

12. Injektor (1, 40) nach einem der Ansprüche 5 bis 11, wobei die fluidische Verbindung zwischen dem Gaszuführrohr (2) und der Öffnung (18, 19, 32, 33) einen oder mehrere Luer-Verbinder (16, 17) aufweist.

13. System (100), das eine Steuereinheit (110), eine Gaszufuhr (120), und eine Flüssigkeitszufuhr (130) aufweist, wobei jede der Gaszufuhr (120) und der Flüssigkeitszufuhr (130) mit einem jeweiligen Gaszuführrohr (2) und Flüssigkeitszuführrohr (3) eines Injektors (1, 40) gemäß einem der vorhergehenden Ansprüche verbunden ist, wobei die Steuereinheit (110) zu folgendem ausgelegt ist:
Aktivieren der Gaszufuhr (120), um das Gas in das Gaszuführrohr (2) zu drücken, vorbei an dem Auswahldurchflusselement (5) bei einem Gasdruck X, und um Gas zu der Abflusskanüle (12) zu führen, und
Wechseln zu einer Zufuhr von Flüssigkeit (130) durch die Abflusskanüle (12) durch das Strömen von Flüssigkeit bis zu dem Auswahldurchflusselement (5) bei einem Flüssigkeitsdruck, der höher als der Gasdruck X ist, und Zuführen von Flüssigkeit zu der Abflusskanüle (12).

14. System (100) nach Anspruch 13, wobei die Steuereinheit ferner ausgelegt ist, um beim Schritt des Aktivierens der Gaszufuhr (120) die Flüssigkeitszufuhr (130) zu aktivieren und Flüssigkeit über das Flüssigkeitszuführrohr (3) hinaus zu drücken, sodass ein Flüssigkeit-Gas-Übergang in dem Flüssigkeitszuführrohr (3) gebildet ist.

15. System (100) nach Anspruch 13 oder Anspruch 14, wobei die Steuereinheit ausgelegt ist, um den Flüssigkeitsdruck und den Gasdruck in jedem Zuführrohr (2, 3) in einem Bereich von 0 bis 200 mmHg zu steuern.

## Revendications

1. Injecteur (1, 40) destiné à être utilisé dans des applications ophtalmiques, ayant un tube d'alimentation en gaz (2) et un tube d'alimentation en liquide (3), les deux tubes d'alimentation étant reliés entre eux au niveau d'un élément de jonction (4, 24) qui est en communication fluidique avec une canule de sortie (12),
**caractérisé en ce qu'**un élément d'écoulement sélectif (5), formé par un filtre hydrophobe, est placé dans le tube d'alimentation en gaz (2) à une distance de l'élément de jonction (4, 24) correspondant à un volume V_{B} du tube d'alimentation en gaz pouvant être rempli de liquide, permettant le passage des gaz et bloquant le passage de liquide.

2. Injecteur (1, 40) selon la revendication 1, l'élément d'écoulement sélectif (5) étant constitué d'une membrane comprenant du polytétrafluoroéthylène (PTFE), la membrane présentant une taille de pores maximale comprise entre 0,2 et 1,0 µm.

3. Injecteur (1, 40) selon la revendication 2, la membrane présentant une épaisseur comprise entre 0,01 et 1 mm.

4. Injecteur (1, 40) selon l'une quelconque des revendications précédentes, où le volume V_{B} est au plus de 1,5 ml, de préférence au plus de 0,5 ml, et plus préférablement au plus de 0,1 ml.

5. Injecteur (1, 40) selon l'une quelconque des revendications précédentes, l'élément d'écoulement sélectif (5) étant placé dans le tube d'alimentation en gaz (2) dans une chambre (8, 25), la chambre (8, 25) ayant un premier segment (13, 26) et un deuxième segment (14, 27), le deuxième segment (14, 27) étant le plus proche de l'élément de jonction (4, 27), chacun du premier segment (13, 26) et du deuxième segment (14, 27) ayant une ouverture (18, 19, 32, 33) dans sa surface, l'élément d'écoulement sélectif (5) séparant la chambre (8, 25) en un premier compartiment situé près du premier segment (13, 26) et un deuxième compartiment situé près du deuxième segment (14, 27), chacun du premier compartiment et du deuxième compartiment étant en connexion fluidique avec le tube d'alimentation en gaz (2) via l'ouverture (18, 19, 32, 33), le liquide dans le deuxième compartiment étant bloqué de pénétrer dans le premier compartiment.

6. Injecteur (1, 40) selon la revendication 5, chacun du premier et du deuxième segment (26, 27) ayant sur sa surface un connecteur (28, 29) s'étendant radialement vers l'ouverture (32, 33) pour recevoir le tube d'alimentation en gaz (2), le tube d'alimentation en gaz (2) étant en connexion fluidique avec le premier compartiment et le deuxième compartiment de la chambre (25) via l'ouverture (32, 33).

7. Injecteur (1, 40) selon l'une quelconque des revendications 5 ou 6, le premier segment (13, 26) et le deuxième segment (14, 27) étant des éléments intégrants de la chambre (8, 25).

8. Injecteur (1, 40) selon l'une quelconque des revendications 5 ou 6, le premier segment (13, 26) et le deuxième segment (14, 27) étant connectés de manière amovible.

9. Injecteur (1, 40) selon l'une quelconque des revendications 5 à 7, l'ouverture (18, 32) dans la surface du premier segment (13, 26) et l'ouverture (19, 33) dans la surface du deuxième segment (14, 27) étant sensiblement alignées le long d'un axe principal du tube d'alimentation en gaz (2).

10. Injecteur (1, 40) selon l'une quelconque des revendications 5 à 9, la chambre (8, 25) ayant un volume de 1 ml, de préférence de 0,3 ml, plus préférablement de 0,1 ml.

11. Injecteur (1, 40) selon l'une quelconque des revendications 5 à 10, la chambre (8, 25) ayant une forme ronde, telle qu'une forme de disque ou une forme sphérique.

12. Injecteur (1, 40) selon l'une quelconque des revendications 5 à 11, la connexion fluidique entre le tube d'alimentation en gaz (2) et l'ouverture (18, 19, 32, 33) comprenant un ou plusieurs connecteurs Luer (16, 17).

13. Système (100) comprenant une unité de contrôle (110), une alimentation en gaz (120) et une alimentation en liquide (130), chacune de l'alimentation en gaz (120) et de l'alimentation en liquide (130) étant connectée respectivement à un tube d'alimentation en gaz (2) et à un tube d'alimentation en liquide (3) d'un injecteur (1, 40) selon l'une quelconque des revendications précédentes, l'unité de contrôle (110) étant adaptée pour :
- activer l'alimentation en gaz (120) pour faire monter du gaz dans le tube d'alimentation en gaz (2), au-delà de l'élément d'écoulement sélectif (5), à une pression de gaz X, et fournir du gaz à la canule de sortie (12), et
- commuter vers une alimentation en liquide (130) à travers la canule de sortie (12) en faisant s'écouler du liquide jusqu'à l'élément d'écoulement sélectif (5) à une pression de liquide supérieure à la pression de gaz X, et en fournissant du liquide à la canule de sortie (12).

14. Système (100) selon la revendication 13, l'unité de contrôle étant en outre adaptée pour, lors de l'étape d'activation de l'alimentation en gaz (120), activer l'alimentation en liquide (130) et faire monter le liquide au-delà du tube d'alimentation en liquide (3) de manière à former une jonction liquide-gaz dans le tube d'alimentation en liquide (3).

15. Système (100) selon la revendication 13 ou la revendication 14, l'unité de contrôle étant adaptée pour contrôler la pression de liquide et la pression de gaz dans chaque tube d'alimentation (2, 3) dans une plage comprise entre 0 et 200 mmHg.
